(19) Europäisches Patentamt
European Patent Office
Office européen des brevets

(11) EP 2 756 842 A1

# (12) EUROPEAN PATENT APPLICATION

(43) Date of publication:
**23.07.2014 Bulletin 2014/30**

(21) Application number: **14151527.0**

(22) Date of filing: **17.01.2014**

(51) Int Cl.:
***A61K 9/51*** *(2006.01)* ***A61K 38/28*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.01.2013 US 201361849057 P**

(71) Applicant: **Nano and Advanced Materials Institute Limited**
**Hong Kong (CN)**

(72) Inventors:
- **Yu, Peter Hoi Fu**
  **Hong Kong (CN)**
- **Chan, Alex Ho Yin**
  **Hong Kong (CN)**
- **Ho, Kwok Ping**
  **Hong Kong (CN)**

(74) Representative: **Kasche, André**
**Kasche & Partner AG**
**Resirain 1**
**8125 Zollikerberg (CH)**

## (54) Synthesis and use of Polyhydroxyalkanoate (PHA) Nanocapsules as a protein carrier

(57) A method of synthesizing PHA nanocapsules as a protein carrier, particularly, the synthesis of PHA nanocapsules synthesized by a modified precipitation/solvent evaporation technique with neutralization. Specifically, the method comprises the steps of sonicating a first solution comprising a triblock PHA copolymer, stirring the first solution into a second solution to give a third solution, the second solution containing a protein, and the second solution being acidic. Further, the method comprises adding the third solution with a neutralizing agent to give a neutralized solution, and sonicating the neutralized solution to form a final solution, wherein the synthesized PHA nanocapsules containing the protein are formed in the final solution. Further, PHA nanocapsules encapsulated with the protein is used by administering the synthesized PHA nanocapsule to an animal in need thereof via the oral cavity.

EP 2 756 842 A1

## Description

### Technical Field

The present application is directed towards a method of synthesizing Polyhydroxyalkanoate (PHA) nanocapsules that encapsulate a protein within them.

### Background

It has been a challenge to find suitable oral delivery methods for some proteins that cannot be administered orally due to their imminent enzymatic degradation and poor mucosal permeability caused due to the highly acidic conditions in the stomach. Encapsulation of peptides and proteins in polymeric nanocapsules has been reported to be a promising way to administer these proteins as the nanocapsules facilitate in increasing the bioavailability of the drug by preventing absorption of the drug in the gastrointestinal tract.

Research has shown that nanoparticles as drug carriers are stable in the gastrointestinal tract and can protect their encapsulated drugs from enzymatic degradation. The physicochemical characteristics, drug release properties and biological behavior of nanoparticles can be easily modified by tailoring their polymeric materials. Moreover, their submicron size and large specific area favor their absorption compared with larger carriers (Rieux *et at.,* 2006), as well as avoiding opsonization and subsequent recognition and phagocytosis by macrophages (Harashima *et al.,* 1994). The use of polymeric nanocapsules to encapsulate therapeutic drugs can protect them from enzymatic degradation (Lowe and Temple, 1994) and can enhance their transmucosal transport (Mathiowitz *et al*., 1997). Encapsulation of such proteins in nanocapsules for oral delivery in an animal has been found to be a promising method of enhancing their bioavailability by protecting the protein from enzymatic degradation in the gastrointestinal tract.

Nanocapsules derived from microbial origin as drug carriers are believed to have a higher degree of biodegradability and biocompatibility than the synthetic capsules, and to be more durable to resist biodegradation in the gastrointestinal tract, which in turn enhances their drug loading and release capabilities in the small intestines. Microbial PHAs are a family of naturally occurring biodegradable and biocompatible polyesters. They are produced by certain microorganisms as intracellular carbon and energy storage polymers. PHAs are attractive materials for drug delivery applications due to their lack of cytotoxic effects upon delivery. Further, PHA nanocapsules are believed to have a higher degree of biodegradability and biocompatibility than synthetic capsules, as they are more resistant to enzymatic degradation in the gastrointestinal tract.

In Chen et al., "Preparation and characterization of biodegradable nanoparticles based on amphiphilic poly(3-hydroxybutyrate)-poly(ethylene glycol)-poly(3-hydroxybutyrate) triblock polymer," European Polymer Journal, pages 2211-2220, (2006), (hereinafter Chen) the authors of the paper discuss the synthesis of PHB-PEG-PHB triblock copolymers and a study of these nanoparticles is performed. Additionally, the reference also discloses a possibility of using these nanoparticles as delivery vehicles for hydrophobic drugs.

Chen teaches the synthesis of nanoparticles using precipitation/solvent evaporation technique, with pyrene as an imitative drug, only to demonstrate that PHA nanoparticles are suitable for drug delivery. However, experimentation has shown that the amount of drug/ protein that is encapsulated in the nanocapsules synthesized by the precipitation/ solvent evaporation technique is very low, and therefore an inefficient method to administer proteins. This is because poor encapsulation efficiency of the protein leads to its low bioavailability, when administered orally. Therefore, a need still exists to deliver proteins orally via nanocapsules wherein the encapsulation efficiency of the protein is high, so that the bioavailability of the protein increases.

### Summary

The present application is directed towards a method of synthesizing PHA nanocapsules as a protein carrier. An object of this application is the synthesis of PHA nanocapsules by a modified precipitation/solvent evaporation technique with neutralization. Specifically, the method comprises the steps of sonicating a first solution comprising a triblock PHA copolymer, stirring the first solution into a second solution to give a third solution, the second solution comprising a protein, and the second solution being acidic. Further, the method comprises neutralizing the third solution with a neutralizing agent to give a neutralized solution, and sonicating the neutralized solution to form a final solution, wherein the synthesized PHA nanocapsules are formed in the final solution.

Another object of the present subject matter is a method of synthesizing PHA nanocapsules as a protein carrier, whereby a high encapsulation efficiency of the protein within the PHA nanocapsules is obtained, so that the bioavailability of the protein increases when the PHA nanocapsules are orally administered.

The present subject matter is also directed towards a PHA nanocapsule synthesized by the method described above.

Another object of the present subject matter is to synthesize optimal PHA nanocapsules for insulin by using a modified precipitation/solvent evaporation technique, with neutralization to enhance the oral bioavailability of the encapsulated insulin.

**Brief Description of the Drawings**

Figure 1 is a flowchart describing the steps of the method of synthesis of PHA nanocapsules
Figure 2 is an illustration of the synthesis of PHA nanoparticles.
Figure 3 is a depiction of $PHB_{16}$-$PEG_{91}$-$PHB_{16}$ as seen under a scanning electron microscope.

**Detailed Description**

All technical and scientific terms used herein have the same meanings as commonly understood by someone ordinarily skilled in the art to which this subject matter belongs. The following definitions are provided for the purpose of understanding the present subject matter and for constructing the appended patent claims.

The term "a" or "an" as used herein includes the singular and the plural, unless specifically stated otherwise. Therefore, the term "a," "an" or "at least one" can be used interchangeably in this application.

Throughout the application, descriptions of various embodiments use the term "comprising"; however, it will be understood by one of skill in the art, that in some specific instances, an embodiment can alternatively be described using the language "consisting essentially of" or "consisting of."

For purposes of better understanding the present teachings and in no way limiting the scope of the teachings, unless otherwise indicated, all numbers expressing quantities, percentages or proportions, and other numerical values used herein, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

"Polyhydroxyalkanoate" or "PHA" as used herein refers to naturally biodegradable and biocompatible polyesters of microbial origin. They are produced by certain microorganisms as intracellular carbon and energy storage polymers.

"Nanocapsules" as described herein are particles that are made out of triblock copolymers having a shell which can be loaded with a protein or drug on a nano-scale.

"Biocompatibility", as used herein refers to the ability of the nanocapsules to perform their function of being a protein carrier without eliciting any undesirable effects in the animal that the nanocapsule is being delivered to.

"Biodegradability" as used herein is the dissolution of the nanocapsules with proteins encapsulated in it within the gastrointestinal tract.

An "animal", as used herein application refers to mammals and cold-blooded animals.

Other terms as used herein are meant to be defined by their well-known meaning in the art.

Figure 1 of the present application illustrates a flowchart of the method of synthesizing PHA nanocapsules encapsulating a protein, so that the PHA nanocapsules function as a protein carrier. The PHA nanocapsules are formed by the precipitation/solvent evaporation technique without the addition of any surfactants, with some modifications.

As shown in Figure 1, at step S100, a first solution is subject to sonication. After sonication, at step S110, the first solution is added dropwise into a second solution, while being stirred. At step S110, a third solution is formed. The third solution, at step S120 is neutralized, with the help of a neutralizing agent. The neutralization at step S130 results in a final solution. The formation of the PHA nanocapsules can be seen in the final solution, as depicted in step S 140 of Figure 1.

In one embodiment, sonication is performed between 20kHz-100kHz with an ultrasonic device.

The first solution of step S 100 comprises a triblock copolymer solution. Further, the first solution also comprises a ketone, such as acetone, where the triblock copolymer solution is dissolved in acetone. The dissolution of the triblock copolymer solution in acetone takes place under sonication, as shown in step S100 of Figure 1.

The triblock copolymer in one embodiment of the method of synthesis is poly(3-hydroxybutarate)-poly(ethylene glycol)-poly(3-hydroxybutarate) (hereinafter PHB-PEG-PHB). PHB-PEG-PHB of microbial origin is a biocompatible and biodegradable compound, and therefore is an ideal choice for the synthesis of nanocapsules as protein carriers. Other triblock copolymers that are well known to a person of skill in the art can also be used. In one embodiment, the triblock copolymer is amphiphilic.

The second solution as shown in step S110 of Figure 1 comprises a protein. In one embodiment of the method of synthesis, the protein is a hormone. By way of non-limiting examples, the protein can be selected from a group

consisting of dynorphin, adrenocorticitropic hormone, thyroid stimulating hormone, luteinizing hormone, growth hormone, atrial natriuretic hormone, antidiuretic hormone, insulin, glucagon, somatostatin, gastrin, cholecystokinin, and combinations thereof. Further, drugs that are known to a person of skill in the art to be absorbed in the small intestine can also be encapsulated in the PHA nanocapsules. In one embodiment, PHA nanocapsules are synthesized where the protein encapsulated within the PHA nanocapsules is insulin.

In one embodiment of the method of synthesis, the protein is soluble in an acidic solution. Consequently, the acid soluble protein is insoluble in a solution that has been neutralized by an acid neutralizing compound. Further, in some embodiments, the protein is soluble in a basic solution. Consequently, the base soluble protein is insoluble in a solution that has been neutralized by a base neutralizing compound.

In one embodiment, the second solution comprises distilled and deionized water. Further, the protein to be encapsulated can be solubilized in distilled and deionized water.

As shown in step S110 of Figure 1, the stirring of the first solution into the second solution gives rise to a third solution. The third solution is subject to neutralization, as shown in step S120 of Figure 1.

In one embodiment, neutralization of the third solution is carried out by using a neutralizing agent. Depending on whether the protein that is being encapsulated is soluble in an acidic or basic solution, an appropriate neutralizing agent is used. For example, if the protein being encapsulated is soluble in acidic solutions, then an acid neutralizing agent is used. In the alternative, if the protein being encapsulated is soluble in basic solutions, a base neutralizing agent is used.

Various neutralizing agents known to a person of skill in the art can be used in this regard. Specific, non-limiting examples of acid neutralizing agents that can be used in this regard are sodium carbonate, calcium carbonate, calcium oxide, magnesium hydroxide, and sodium bicarbonate. Further, non-limiting examples of base neutralizing agents that can be used as hydrocholic acid, sulphuric acid, and carbonic acid.

The neutralization step of the method of synthesis herein described is of particular importance because, during this step, the acid soluble protein becomes insoluble due to the effect of the neutralizing agent on the third solution. During experimentation, it was unexpectedly found that the change in solubility of the protein in the neutralized solution increases the encapsulation efficiency of the protein within the PHA nanocapsules. Hence, due to an increase in the encapsulation efficiency, the bioavailability of the protein, when administered to an animal increases. Further, the PHA nanocapsules function particularly well as protein carriers because PHA nanocapsules are biocompatible with animals and have a high degree of biodegradability in the gastrointestinal tract, thereby providing an efficient and sustained release of the protein.

The neutralization step S130 of Figure 1 results in a final solution, where the PHA nanocapsules are formed within the solution, as discussed in step S140 of Figure 1. In one embodiment, PHA nanocapsules are synthesized with the protein encapsulated within the nanocapsule.

Further, in another embodiment, the PHA nanocapsules are about 50-1000 nm in size. In another embodiment, the synthesized PHA nanocapsules are about 100-500 nm in size.

In other embodiments, the PHA nanocapsules are synthesized in such a way that the PHA nanocapsules have a hydrophilic outer shell and a hydrophobic core. Alternative embodiments are present, where the protein is encapsulated in the hydrophobic core. Such PHA nanocapsules have been found to be particularly useful in the delivery of proteins because the hydrophilic outer shell prevents the PHA nanocapsules from enzymatic degradation in an animal's gastrointestinal tract, thereby facilitating efficient delivery of the protein.

Figure 2 shows an illustration of the process explained in Figure 1 above. In one embodiment, Polyethylene glycol (PEG) is combined with BL monomer to form a first solution comprising PHB-PEG-PHB triblock copolymer, and stannous octoate is added as a solution in dried dicholoromethane, as shown in step 200 of Figure 2. Further, the resulting PHB-PEG-PHB triblock copolymer, as shown in step S210, is subject to sonication. Additionally, the PHB-PEG-PHB triblock copolymer is stirred into a second solution to give a third solution. The second solution comprises the protein to be encapsulated within the PHB-PEG-PHB nanocapsule.

Upon neutralization of the third solution with a neutralizing agent, a neutralized solution is formed as shown in step S220 of Figure 2. Further sonication gives rise to the self-assembly of amphiphilic PHB-PEG-PHB triblock copolymers in an aqueous medium (or the final solution) with the protein encapsulated within, as shown in step S230 of Figure 2. Further, the self-assembled PHB-PEG-PHB triblock copolymers are made of a hydrophilic outer shell comprising PEG, and a hydrophobic central core comprising PHB.

Step S240 depicts a SEM micrograph of the PHB-PEG-PHB nanoparticles for Sample P11, as shown in Table 1, below.

Table 1 discusses the various samples of PHB-PEG-PHB structures and sizes of the PHA nanocapsules.

Table 1: Sizes of Nanocapsules that can be used

| Sample name | Structure ($PHB_X$-$PEG_y$-$PHB_X$) | Size (nm) |
|---|---|---|
| P11 | $PHB_{11}$-$PEG_{91}$-$PHB_{11}$ | 171.4 |
| P15 | $PHB_{15}$-$PEG_{91}$-$PHB_{15}$ | 124.4 |
| P16 | $PHB_{16}$-$PEG_{91}$-$PHB_{16}$ | 180.4 |
| P17 | $PHB_{17}$-$PEG_{91}$-$PHB_{17}$ | 82.5 |
| P22 | $PHB_{22}$-$PEG_{91}$-$PHB_{22}$ | 1893.5 |
| P27 | $PHB_{27}$-$PEG_{91}$-$PHB_{27}$ | 189.3 |
| P40 | $PHB_{40}$-$PEG_{91}$-$PHB_{40}$ | 275.8 |
| P42 | $PHB_{42}$-$PEG_{91}$-$PHB_{42}$ | 248 |
| P43 | $PHB_{43}$-$PEG_{91}$-$PHB_{43}$ | 248.6 |
| P52 | $PHB_{52}$-$PEG_{91}$-$PHB_{52}$ | 321.6 |

Further, Figure 3 discloses a SEM micrograph of the PHB-PEG-PHB nanoparticles for Sample P16 as tabulated in Table 1.

In one embodiment, PHA nanocapsules for insulin are synthesized by the following procedure: 1) a triblock copolymer solution in dissolved in acetone, forming a first solution, under sonication; 2) the first solution is added dropwise to a second solution containing distilled and deionized water (DD $H_2O$) and 0.5ml or 0.75ml of insulin (0.1g/ml in 0.1M HCl) under stirring for 15 min to give a third solution; 3) a volume of 275ul or 413ul of 0.1M sodium carbonate, which acts as a neutralizing agent is added to neutralize the third solution during the formation of PHA nanocapsules for insulin; 4) The neutralized solution was then subject to ultrasonic for another 15 min to give a final solution.

In one embodiment, the synthesized PHA nanocapsules are used in a method of treating a disease or a condition in an animal, where the PHA nanocapsules are administered to an animal in need thereof via the oral cavity. The diseases that can be treated will vary based on the protein or drug encapsulated within the nanocapsules. For example, if the protein or drug encapsulated within the nanocapsules is insulin, the nanocapsules can be used to treat any disease or condition treatable by insulin, such as diabetes. In this vein, any disease or condition treatable by dynorphin, adrenocorticitropic hormone, thyroid stimulating hormone, luteinizing hormone, growth hormone, atrial natriuretic hormone, antidiuretic hormone, insulin, glucagon, somatostatin, gastrin, cholecystokinin, and combinations thereof is contemplated as being treated by the nanocapsules described herein. Specifically, diseases such as Addison's disease, hypothyroidism, hypoglycemia can be treated by the administration of nanocapsules. Further, these nanocapsules can also be used in fertility treatments for females and males.

Nanocapsules synthesized by the claimed method can also be used to encapsulate other active ingredients instead of proteins. Active ingridients such as peptides, proteins, oligonucleotides, polynucleotides, and plasmids, may be incorporated in the nanocapsules. Peptides may have a wide range of sizes, such as from a dipeptide to a peptide containing 1,000 amino acids. The active agents may also be antibacterial agents, anti-inflammatory and anticancer agents, antidepressants, analgesics, or local anesthetics. Examples of such active agents, include aminoglycoside antibiotics, quinoline antibiotics, antimicrobial agents, antifungal agents and antiviral agents, lidocaine, bupivacaine, non-steroidal anti-inflammatory drugs, steroids, morphine and narcotic agents, antidepressants, risperidol and other drugs intend to improve mental behavior, risporidol, tetracycline, chlorhexidin, and aminocycline. In one preferred embodiment the active agent is a drug from the family of taxans, platin derivatives or antimetabolites.

**Examples**

Synthesis of the PHA Triblock Polymers

In one embodiment, Polyethylene glycol (PEG) is combined with BL monomer to form a first solution comprising PHB-PEG-PHB triblock copolymer. The purified BL monomer and PEG were placed in a dried polymerization flask with appropriate ratio, and stannous octoate [$Sn(Oct)_2$] in desired concentrations was added as a solution in dried $CH_2Cl_2$. The reaction is shown below.

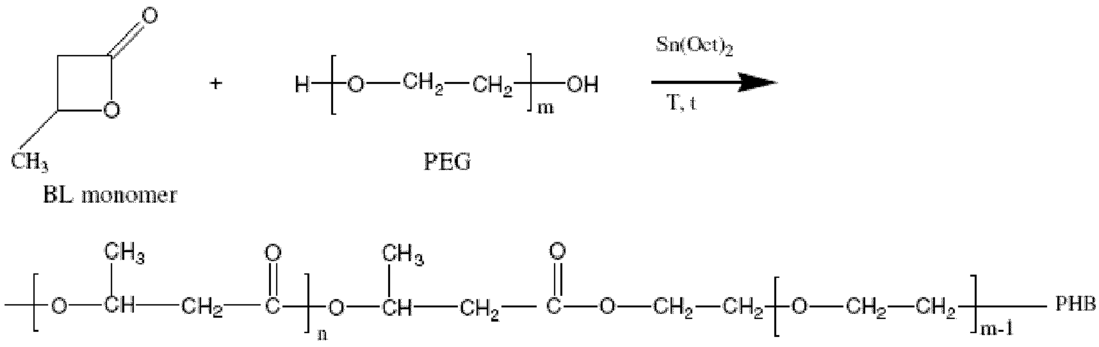


BL monomer

PEG

PHB-PEG-PHB

The reactants were mixed and then dried under reduced pressure at 40 °C for 1 hour to completely remove $CH_2Cl_2$. The reactions were carried out under nitrogen atmosphere at desired temperatures. After specified reaction time, the reacted products were repeatedly dissolved in chloroform, and then precipitated in hexane several times to remove the unreacted BL monomer. The isolated products were dried in vacuum at 40 °C for 48 hours.

Further, the triblock copolymer solution was dissolved in 1 ml acetone by sonication. The solution was added dropwise to distilled and deionized water (DD $H_2O$) containing 0.5ml/0.75ml of insulin (0.1g/ml in 0.1M HCl) under stirring for 15 min, 275ul/413ul of 0.1M sodium carbonate was added, followed by ultrasonic situation for 15 min. Acetone was then removed under lower pressure and ultrasonic situation for 3 hours using vacuum oven. All dispersions were filtered using disposable 0.45 μm Millipore filters, without significant effect on the particle yield or size distribution.

Samples P16 and P43 were synthesized in the method described above. The encapsulation efficiency of insulin was conducted, where various concentrations of insulin was encapsulated into the nanocapsules. Insulin-loaded nanocapsules solution was centrifuged and the supernatant was collected for HPLC analysis to determine the efficiency of insulin encapsulation. The association efficiency and loading capacity for each sample were calculated based on the formulas below.

$$\text{Association efficiency (AE)} = \frac{\text{Total amount of insulin} - \text{Free insulin in supernatant}}{\text{Total amount of insulin}} \times 100$$

$$\text{Loading capacity (LC)} = \frac{\text{Total amount of insulin} - \text{Free insulin in supernatant}}{\text{Total weight of nanoparticles}} \times 100$$

Table 2: Association efficiency and Loading capacity of nanocapsules encapsulated with Insulin as synthesized by method above

| | Sample | Area (mAU*s) | Insulin Conc. (μg/ml) | Weight of nanocapsule formed | AE (%) | LC(%)(old) | LC(%)(new) |
|---|---|---|---|---|---|---|---|
| P16 | Supernatant from 50 μg/ml insulin-loaded nanocapsule | N/A | N/A | 0.0004 | 100 | 5 | 125 |
| P16 | Supernatant from 50 μg/ml insulin-loaded nanocapsule after acid wash | 15.2 | 15.36 | 0.0004 | 69.3 | 3.46 | 86.6 |
| P16 | Supernatant from 75 μg/ml insulin-loaded nanocapsule | N/A | N/A | 0.00106 | 100 | 7.5 | 47.2 |

(continued)

| | Sample | Area (mAU*s) | Insulin Conc. (μg/ml) | Weight of nanocapsule formed | AE (%) | LC(%)(old) | LC(%)(new) |
|---|---|---|---|---|---|---|---|
| P16 | Supernatant from 75 μg/ml insulin-loaded nanocapsule after acid wash | 48 | 54.17 | Failed | 27.78 | 2.1 | ? |
| P43 | Supernatant from 50 μg/ml insulin-loaded nanocapsule | N/A | N/A | 0.00094 | 100 | 5 | 53.2 |
| P43 | Supernatant from 50 μg/ml insulin-loaded nanocapsule after acid wash | 22.4 | 22.31 | 0.00046 | 44.6 | 2.2 | 60.2 |

As can be seen from Table 2 above, the association efficiency and the loading capacity of the nanocapsules is particularly high for larger quantities of insulin such as 50 μg/ml and 75 μg/ml. This can be further understood by comparing Table 2 to Table 3 below, where Table 3 indicates the association efficiency and loading capacity of nanocapsules that are synthesized by the precipitation solvent evaporation method, without the neutralization step.

Table 3: Association efficiency and Loading capacity of nanocapsules encapsulated with Insulin synthesized by the precipitation solvent evaporation technique (without neutralization)

| Sample | Area (mAU*s) | Retention time(min) | Insulin Concentration (μg/ml) | Association Efficiency (%) | Loading Capacity (%) |
|---|---|---|---|---|---|
| Supernatant from 10 μg/ml insulin-loaded nanoparticle | N/A | N/A | N/A | 100 | 0.2 |
| Supernatant from 50 μg/ml insulin-loaded nanoparticle | 605.24652 | 13.082 | 42.27 | 15.46 | 0.02 |
| | 608.42615 | 13.114 | 42.50 | 15.00 | 0.00141 |
| Supernatant from 100 μg/ml insulin-loaded nanoparticle | 1456.52905 | 13.069 | 103.81 | 0 | 0.00214 |
| | 1426.42493 | 13.104 | 101.64 | 0 | 0 |

On comparing Tables 2 and 3 above, it can be seen that while the association efficiency of nanocapsules synthesized by the precipitation solvent evaporation technique (without neutralization) for 10 μg/ml insulin-loaded nanoparticle is very high, the association efficiency sharply decreases when the amount of insulin that is to be encapsulated increases to 50 μg/ml and 100μg/ml. However, in Table 2, it can be seen that even though higher amounts of insulin is encapsulated in the nanocapsules that are synthesized using the method described above, (with the neutralization step), the association efficiency and loading capacity of the nanocapsules is significantly higher.

With the information contained herein, various departures from precise descriptions of the present subject matter will be readily apparent to those skilled in the art to which the present subject matter pertains, without departing from the spirit and scope of the below claims. The present subject matter is not considered limited in scope to the procedures, properties, or components defined, since the preferred embodiments and other descriptions are intended only to be illustrative of particular aspects of the presently provided subject matter. Indeed, various modifications of the described modes for carrying out the present subject matter, which are obvious to those skilled in chemistry, or biochemistry, or related fields are intended to be within the scope of the following claims.

## Claims

**1.** A method of synthesizing PHA nanocapsules as a protein carrier comprising the steps:

- sonicating a first solution comprising a triblock PHA copolymer;
- stirring the first solution into a second solution to give a third solution, the second solution containing a protein;
- adding the third solution with a neutralizing agent to give a neutralized solution; and
- sonicating the neutralized solution to form a final solution;

wherein the synthesized PHA nanocapsules containing the protein are formed in the final solution.

**2.** The method of claim 1, wherein the triblock PHA copolymer is PHB-PEG-PHB.

**3.** The method of claim 1, wherein the first solution further comprises a ketone, preferably acetone.

**4.** The method of claim 1, wherein the second solution comprises distilled and deionized water.

**5.** The method of claim 1, wherein the protein is a hormone, preferably a hormone selected from the group consisting of dynorphin, adrenocorticitropic hormone, thyroid stimulating hormone, luteinizing hormone, growth hormone, atrial natriuretic hormone, antidiuretic hormone, insulin, glucagon, somatostatin, gastrin and cholecystokinin, more preferably insulin.

**6.** The method of claim 1, wherein the neutralizing agent is sodium carbonate.

**7.** The method of claim 1, wherein the protein is soluble in an acidic or in a basic solution.

**8.** The method of claim 1, wherein the protein is insoluble in the neutralized solution neutralized by an acid neutralizing agent.

**9.** The method of claim 1, wherein the protein is insoluble in the neutralized solution neutralized by a base neutralizing agent.

**10.** The method of claim 1, wherein the synthesized PHA nanocapsule has a hydrophilic outer shell and a hydrophobic core.

**11.** The method of claim 10, wherein the protein is in the hydrophobic core.

**12.** The method of claim 2, wherein the triblock PHA copolymer is amphiphilic.

**13.** A PHA nanocapsule synthesized according to the method of claim 1, wherein the PHA nanocapsule preferably ranges from 100-500 nm in size.

**14.** A method of synthesizing PHA nanocapsules as an insulin carrier by a modified precipitation/solvent evaporation technique comprising neutralizing a solution comprising a triblock PHB-PEG-PHB copolymer, a protein, and a ketone with a neutralizing agent to form self-assembled PHA nanocapsules with insulin encapsulated within the PHA nanocapsules.

**15.** A PHA nanocapsule synthesized according to the method of claim 14, wherein the PHA nanocapsule preferably ranges from 100-500 nm in size.

FIG. 1

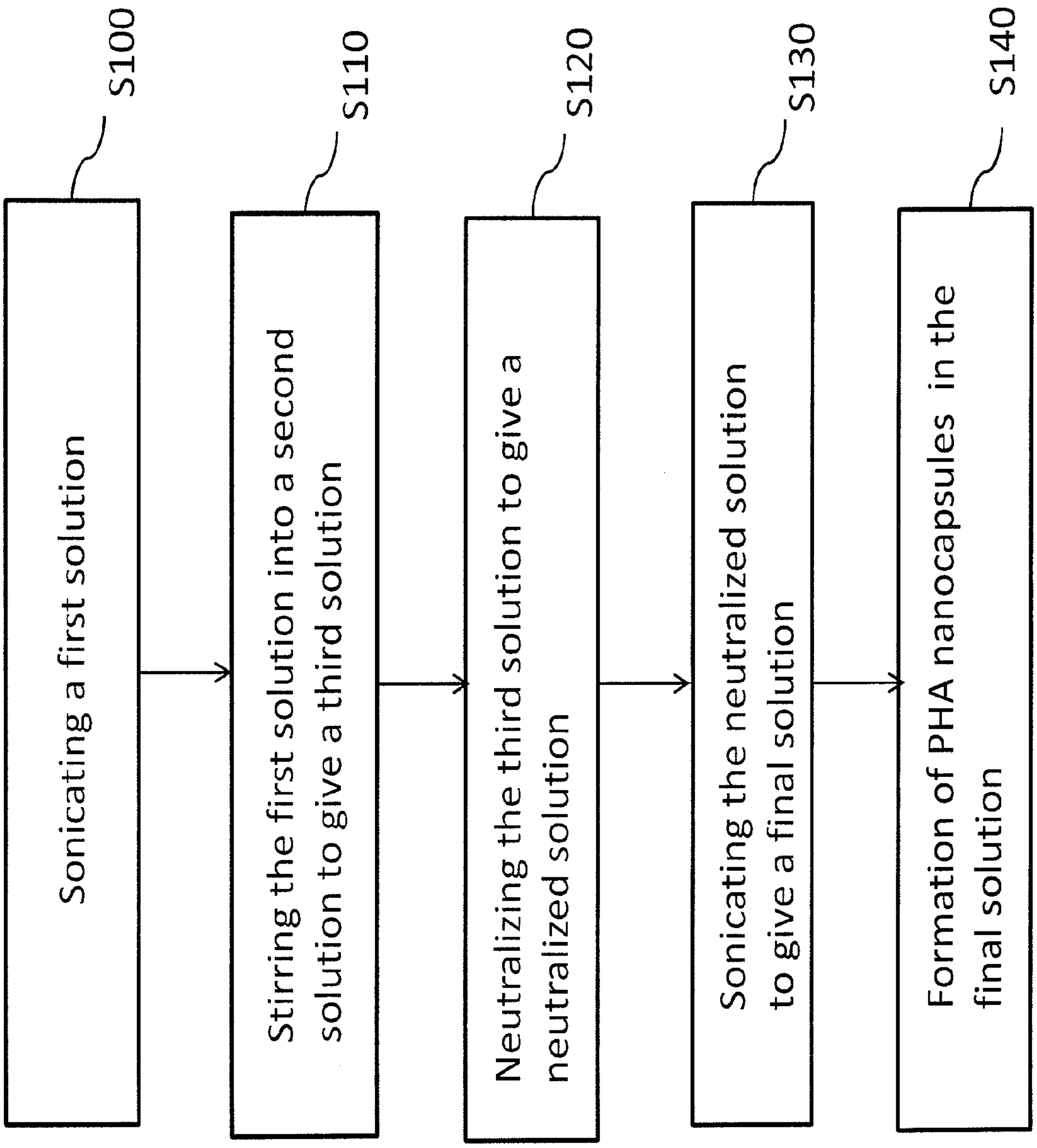
S100
Sonicating a first solution
S110
Stirring the first solution into a second solution to give a third solution
S120
Neutralizing the third solution to give a neutralized solution
S130
Sonicating the neutralized solution to give a final solution
S140
Formation of PHA nanocapsules in the final solution

FiG. 2

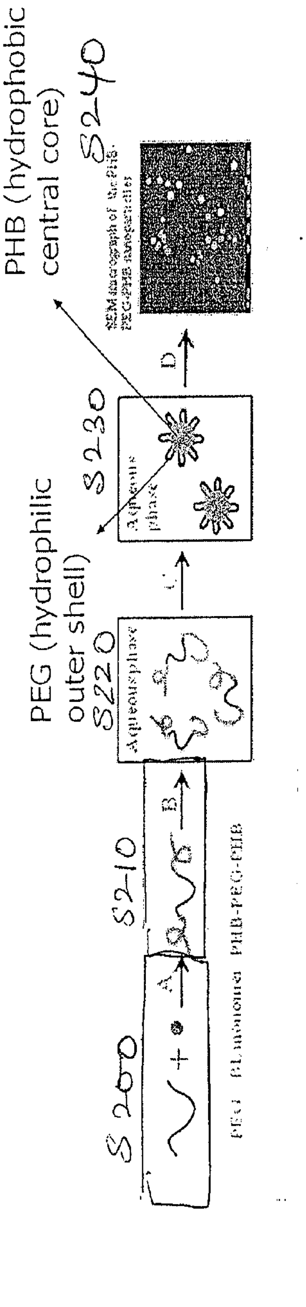
S200
S210
S220
S230
S240
A
B
C
D
PEG (hydrophilic outer shell)
PHB (hydrophobic central core)
PHB-PEG-PHB

FIG. 3

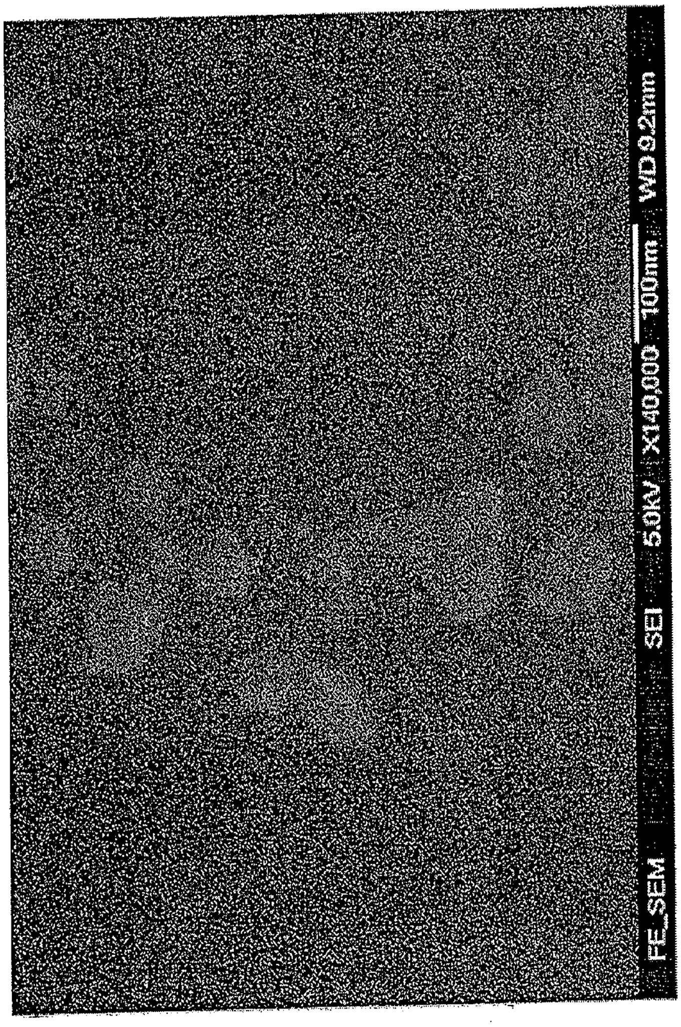


$PHB_{16}$-$PEG_{91}$-$PHB_{16}$

Europäisches Patentamt
European Patent Office
Office européen des brevets

# EUROPEAN SEARCH REPORT

Application Number
EP 14 15 1527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHEN C ET AL: "Preparation and characterization of biodegradable nanoparticles based on amphiphilic poly(3-hydroxybutyrate)-poly(ethylene glycol)-poly(3-hydroxybutyrate) triblock copolymer", EUROPEAN POLYMER JOURNAL, PERGAMON PRESS LTD. OXFORD, GB, vol. 42, no. 10, 1 October 2006 (2006-10-01), pages 2211-2220, XP028029849, ISSN: 0014-3057, DOI: 10.1016/J.EURPOLYMJ.2006.07.001 [retrieved on 2006-10-01] * abstract * * page 2213, left-hand column * ----- | 1-15 | INV. A61K9/51 A61K38/28 |
| Y | Elham Khodaverdi ET AL: "Preparation and characterisation of PLGA-PEG-PLGA nanospheres prepared with a new thermogelling method for insulin delivery", , 1 January 2013 (2013-01-01), XP055122439, Retrieved from the Internet: URL:http://jocpr.com/vol5-iss10-2013/JCPR-2013-5-10-311-319.pdf [retrieved on 2014-06-10] * abstract * * page 313, lines 3-12 * ----- | 1-15 | |
| | -/-- | | TECHNICAL FIELDS SEARCHED (IPC) |
| | | | A61K |

The present search report has been drawn up for all claims

2

| Place of search | Date of completion of the search | Examiner |
|---|---|---|
| Munich | 13 June 2014 | Vázquez Lantes, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 15 1527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YOUNG MIN KWON ET AL: "Biodegradable Triblock Copolymer Microspheres Based on Thermosensitive Sol-Gel Transition", PHARMACEUTICAL RESEARCH, vol. 21, no. 2, 1 February 2004 (2004-02-01), pages 339-343, XP055122539, ISSN: 0724-8741, DOI: 10.1023/B:PHAM.0000016248.30579.2f<br>* abstract *<br>* page 340, left-hand column *<br>----- | 1-15 | |

TECHNICAL FIELDS SEARCHED (IPC)

The present search report has been drawn up for all claims

2

| Place of search | Date of completion of the search | Examiner |
|---|---|---|
| Munich | 13 June 2014 | Vázquez Lantes, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHEN et al.** Preparation and characterization of biodegradable nanoparticles based on amphiphilic poly(3-hydroxybutyrate)-poly(ethylene glycol)-poly(3-hydroxybutyrate) triblock polymer. *European Polymer Journal,* 2006, 2211-2220